(19) Europäisches Patentamt
European Patent Office
Office européen des brevets

(11) Veröffentlichungsnummer: **0 096 288**
**B1**

# (12) EUROPÄISCHE PATENTSCHRIFT

(45) Veröffentlichungstag der Patentschrift: **21.08.85**

(51) Int. Cl.⁴: **C 25 B 3/04**, C 07 H 19/20

(21) Anmeldenummer: **83105114.9**

(22) Anmeldetag: **24.05.83**

(54) **Verfahren zur elektrochemischen Hydrierung von Nicotin-amidadenin-dinucleotid.**

(30) Priorität: **05.06.82 DE 3221339**

(43) Veröffentlichungstag der Anmeldung: **21.12.83 Patentblatt 83/51**

(45) Bekanntmachung des Hinweises auf die Patenterteilung: **21.08.85 Patentblatt 85/34**

(84) Benannte Vertragsstaaten: **CH DE FR GB IT LI**

(56) Entgegenhaltungen:
**CHEMICAL ABSTRACTS, Band 97, Nr. 21, 22. November 1982, Seite 436, Nr. 178215q, Columbus, Ohio, USA, R. WIENKAMP et al.: "Indirect electrochemical processes. Pt. 13. Indirect electrochemical regeneration of NADH by using a bipyridine-rhodium (I) complex as electron carrier"**
**CHEMICAL ABSTRACTS; Band 89, Nr. 25., 18. Dezember 1978, Seite 187, Nr. 210588m, Columbus, Ohio, USA, M.A. JENSEN et al.: "Effect of Lewis acids on the electrochemical reduction of nicotinamide adenine dinucleotide"**

(73) Patentinhaber: **BASF Aktiengesellschaft, Carl-Bosch-Strasse 38, D-6700 Ludwigshafen (DE)**

(72) Erfinder: **Steckhan, Eberhard, Dr., Jungholzweg 26, D-5309 Meckenheim (DE)**
Erfinder: **Wienkamp, Rainer, Am alten Schuetzenhof 26, D-4400 Muenster (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

**Beschreibung**

Diese Erfindung betrifft ein Verfahren zur elektrochemischen Hydrierung von Nicotinamidadenin-dinucleotid ($NAD^{\oplus}$).

$NAD^{\oplus}$ spielt bekanntlich im Organismus bei vielen unter Dehydrierungen und Hydrierungen verlaufenden enzymatischen Prozessen die Rolle des Wasserstoffüberträgers. Bei Redoxreaktionen reagiert das Coenzym $NAD^{\oplus}$/NADH dabei in folgender Weise:

$$NAD^{\oplus} + 2\,H = \rightarrow NADH + H^{\oplus}.$$

Zahlreiche Enzyme und Mikroorganismen sind in der Lage, in Gegenwart von NADH die Reduktion ungesättigter Verbindungen zu bewirken. Diese Reduktionen, bei denen NADH wie beschrieben als Coenzym wirkt, haben bei einer großen Substratbreite den Vorteil einer hohen Stereospezifität. Es ist deshalb wünschenswert, diese vorteilhafte Wirkungsweise für eine gewerblich anwendbare Reduktion ungesättigter Verbindungen, insbesondere zur Herstellung chiraler Moleküle heranzuziehen. Eine derartige Möglichkeit läßt sich jedoch nur dann wirtschaftlich realisieren, wenn das bei der Reduktion entstandene $NAD^{\oplus}$ auf einfache Weise zu NADH regeneriert werden kann.

Reduziert man $NAD^{\oplus}$ elektrochemisch zu NADH, so werden bei −1,1 Volt, bezogen auf die Kalomelelektrode, im wesentlichen enzyminaktive Dimere gebildet. Da auch bei einem sehr negativen Potential von −1,8 Volt, bezogen auf die Kalomelelektrode, das NADH neben unwirksamen Dimeren und Isomeren nur in 50%iger Ausbeute gebildet wird, kommt die direkte elektrochemische Reduktion für die Regenerierung von $NAD^{\oplus}$ praktisch nicht in Betracht.

Man hat deshalb schon vorgeschlagen, das $NAD^{\oplus}$ durch eine indirekte elektrochemische Reduktion zu regenerieren, bei der man die Elektrolyse in Gegenwart eines Elektronenüberträgers und eines zusätzlichen Enzyms, das die Elektronenübertragung auf das Coenzym $NAD^{\oplus}$ katalysiert, durchführt.

So werden in Angew. Chem., 98 (1981), 897, in J. Org. Chem., 46 (1981), 4622, sowie in J. Org. Chem., 46 (1981), 4100 Verfahren beschrieben, bei denen man als Elektronenüberträger Methylviologen oder trans-4,5-Dihydroxy-1,2-dithian verwendet. Diese Methoden haben jedoch den Nachteil, daß die zur Katalyse der Elektronenübertragung notwendigen Enzyme empfindlich, schwer zu reinigen und nur mit aufwendigen Methoden zu isolieren sind.

Es wurde nun gefunden, daß man die elektrochemische Hydrierung von Nicotinamidadenin-dinucleotid ($NAD^{\oplus}$) zu NADH in wäßriger Lösung und in Gegenwart eines Elektronenüberträgers erheblich vorteilhafter durchführen kann, wenn man als Elektronenüberträger Metallkomplexverbindungen verwendet.

Besonders geeignet sind Metallkomplexverbindungen mit einem Reduktionspotential nicht negativer als −1,3 Volt, vorzugsweise nicht negativer als −0,9 Volt, bezogen auf die Ag/AgCl-Elektrode. Metallkomplexverbindungen der genannten Art enthalten als Zentralatom z. B. $Rh^{I}$, $Rh^{III}$, $Ru^{I}$, $Ru^{III}$, $Ir^{I}$, $Ir^{III}$, $Fe^{II}$, $Fe^{0}$, $Ni^{II}$, $Ni^{0}$, $Co^{III}$, $Co^{I}$ und als Liganden z. B. 2,2'-Bipyridin, 4,4'-Dimethyl-2,2'-bipyridin, 1,10-Phenanthrolin, 2,2',6',2-Terpyridin, Tetraazamakrocyclen, ein Porphyrin, ein Phthalocyanin oder NO.

Von diesen Metallkomplexverbindungen, von denen solche mit organischen Liganden bevorzugt sind, seien im einzelnen folgende Verbindungen genannt:

Aquocobalamina,
$[Rh(bipy)_3]^{3\oplus} X_3^{\ominus}$, $[Rh(bipy)_2]^{3\oplus} X_3^{\ominus}$,
$[Rh(bipy)_2(H_2O)_2]^{3\oplus} X_3^{\ominus}$, $[Ni(PPh_3)_2]^{2\oplus} X_2^{\ominus}$,
$[Rh(bipy)_2(H_2O)]^{\oplus} X^{\ominus}$, $[Ru(bipy)_3]^{3\oplus} X_3^{\ominus}$,
$[Rh(bipy)_2(OH)_2]^{\oplus} X^{\ominus}$, $[Fe(NO)_2Cl]_2$,
$[Rh(bipy)(H_2O)]^{\oplus} X^{\ominus}$, $[Co(NO)_2Br]_2$,

in denen X ein Anion, z. B. Cl bedeutet.

Das neue elektrochemische Verfahren wird vorzugsweise in einer geteilten Zelle bei Temperaturen bis zu 70°C, zweckmäßig bei 10 bis 50°C und einem pH von 5 bis 10 durchgeführt. Als Elektroden wird ein für Elektrosynthesen übliches Elektrodenmaterial verwendet. So sind z. B. Kathoden aus Metallen, wie Blei, Kupfer, Eisen und Nickel oder Graphit und Anoden aus Platin oder Graphit geeignet. Die Stromdichten betragen z. B. 1 bis 100 $mA/cm^2$. Das Kathodenpotential beträgt z. B. bis zu −1,3 Volt und liegt vorzugsweise zwischen −0,5 bis −0,9 Volt, bezogen auf die Ag/AgCl Elektrode:

Die Elektrolyse wird in wäßriger Lösung durchgeführt, wobei die Lösungen z. B. auch Lösungsvermittler, das sind mit Wasser mischbare organische Lösungsmittel, z. B. Alkohole, wie Methanol und Ethanol oder Ether, wie Dioxan oder Dimethoxyethan enthalten können.

Beispielsweise wird die Umsetzung in einer wäßrigen Lösung von Tris-HCl-Puffer durchgeführt, das sind Lösungen von Tris-(hydroxymethyl)-aminoethan, die durch Zugabe von Salzsäure auf den gewünschten pH-Wert eingestellt werden.

Man führt die elektrochemische Hydrierung von $NAD^{\oplus}$ mit Hilfe der genannten Elektronenüberträger zweckmäßig in Gegenwart der ungesättigten Verbindung, die durch das regenerierte NADH

reduziert werden soll, sowie in Gegenwart solcher Enzyme durch, welche die Reduktion der ungesättigten Verbindungen durch NADH katalysieren.

Geeignete ungesättigte Verbindungen sind z. B. Ketone, wie Cyclohexanon, Methyl oder Ethylcyclohexanon, 2-Norbornanon, Bicyclo[3.2.1]-2-octanon oder Acetophenon, sowie Aldehyde, Ketocarbonsäuren oder Verbindungen mit —C=C-Doppelbindungen.

Geeignete Enzyme sind z. B. alle bekannten NADH- und NADPH (Nicotinamidadenin-dinucleotid-phosphat)-abhängigen Oxido-Reduktasen, das sind Alkoholdehydrogenasen, wie HLADH (Horse-liver-alkohol-dehydrogenase), oder Aminosäuredehydrogenasen.

Nach dem erfindungsgemäßen Verfahren läßt sich die elektrochemische Hydrierung von $NAD^{\oplus}$ zu NADH besonders vorteilhaft durchführen. So wird z. B. die Bildung von Dimeren des $NAD^{\oplus}$ weitgehend verhindert. Außerdem kann man das Verfahren auch in Abwesenheit von Enzymen durchführen, die man bei den bisher bekannten Methoden zugesetzt hat, um die Elektronenübertragung auf das $NAD^{\oplus}$ zu katalysieren.

In den folgenden Beispielen wird das erfindungsgemäße Verfahren am Beispiel der indirekten elektrochemischen Reduktion von $NAD^{\oplus}$ zu NADH mit Hilfe von $Rh(bipy)_2^{\oplus}$ als Elektronenüberträger veranschaulicht, wobei als zu reduzierende Verbindung Cyclohexanon verwendet wird, das in Gegenwart des Enzyms HLADH (Horse-liver-alkohol-dehydrogenase) in Cyclohexanol übergeführt wird. Die dabei im Kathodenraum stattfindenden Reaktionen sind aus dem folgenden Schema ersichtlich:

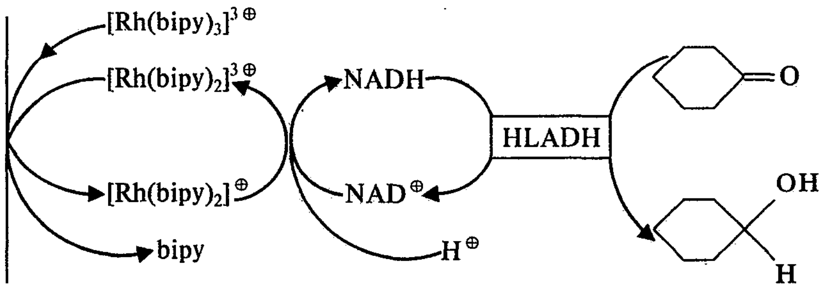


Kathode

Der Elektronenüberträger Tris[2,2'-bipyridyl]-rhodium-trichlorid geht dabei entsprechend der folgenden Gleichung durch Aufnahme von Elektronen an der Kathode in das Bis[2,2'-bipyridyl]-rhodiumchlorid über

$$2\,e^{\ominus} + [Rh(bipy)_3]^{3\oplus}\ \ Cl_3^{\ominus} \longrightarrow [Rh(bipy)_2]^{\oplus}\ \ Cl^{\ominus} + bipy$$

## Beispiele

Die Elektrolyse wurde in einer geteilten Elektrolysezelle mit einer Graphit-Kathode der Oberfläche 6 $cm^2$, einer Platin-Anode und einer Glasfritte als Diaphragma bei Raumtemperatur, einem pH von 9 und einem Potential von —950 mV, bezogen auf eine Ag/AgCl-Elektrode durchgeführt. Als Referenz-Elektrode diente eine Ag/AgCl-Elektrode in gesättigtem wäßrigen Kaliumchlorid.

Als Anolyt wurde eine 0,1molare Lösung von Tris-HCl-Puffer in Wasser verwendet, die mit konzentrierter Salzsäure auf einen pH-Wert von 9 eingestellt wurde. Der Katholyt enthielt als Stammlösung die gleiche Lösung wie der Anolyt sowie die in der folgenden Tabelle angegebenen Mengen an $Rh(bipy)_3Cl_3$, $NAD^{\oplus}$, Cyclohexanon und HLADH.

Die Elektrolyseapparatur wurde vor Zugabe des Cyclohexanons mit Argon gespült und während der Elektrolyse unter einer Argonatmosphäre belassen. Die Stromstärke betrug anfänglich 10 bis 20 mA.

Bei den Beispielen 1 und 2 wurde diskontinuierlich gearbeitet, d. h. es wurde erst nach der indirekten elektrochemischen Hydrierung des $NAD^{\oplus}$ zu NADH das Enzym HLADH und Cyclohexanon zum Katholyten gegeben und anschließend das Cyclohexanol bestimmt. Bei der kontinuierlichen Arbeitsweise gemäß den Beispielen 3, 4 und 5 wurde die Elektrolyse in Gegenwart von HLADH und von Cyclohexanon durchgeführt und das Cyclohexanol gaschromatographisch bestimmt.

Die Einsatzzahlen und Ergebnisse sind aus der folgenden Tabelle ersichtlich:

Tabelle

| Beispiel | $Rh(bipy)_3Cl_3$ mg (mmol) | $NAD^+$ mg (mmol) | Cyclohexanon mg (mmol) | HLADH mg (mmol) | Cyclohexanol mg (mmol) | Anzahl der Cyclen $NAD^+/NADH$ | $Rh^{3+}/Rh^+$ |
|---|---|---|---|---|---|---|---|
| 1 | 384 (0,5) | 332 (0,5) | 59 (0,6) | 2,0 ($2,4 \times 10^{-5}$) | 25 (0,25) | 0,5 | 0,5 |
| 2 | 230 (0,3) | 398 (0,6) | 73,5 (0,75) | 2,0 ($2,4 \times 10^{-5}$) | 29 (0,29) | 0,5 | 0,5 |
| 3 | 115 (0,15) | 100 (0,15) | 96 (0,98) | 1,1 ($1,3 \times 10^{-5}$) | 33 (0,33) | 2,2 | 2,2 |
| 4 | 307 (0,4) | 133 (0,2) | 171 (1,74) | 3,9 ($4,6 \times 10^{-5}$) | 42 (0,42) | 2,1 | 1,0 |
| 5 | 192 (0,25) | 66,5 (0,1) | 110 (1,12) | 2,0 ($2,4 \times 10^{-5}$) | 29 (0,29) | 2,9 | 1,1 |

(Ein Cyclus ist jeweils abgeschlossen, wenn das eingesetzte $NAD^{\oplus}$ einmal zum NADH regeneriert wurde.)

## Patentansprüche

1. Verfahren zur elektrochemischen Hydrierung von Nicotinamid-adenin-dinucleotid ($NAD^{\oplus}$) zu NADH in wäßriger Lösung und in Gegenwart eines Elektronenüberträgers, dadurch gekennzeichnet, daß man als Elektronenüberträger Metallkomplexverbindungen verwendet.

2. Verfahren nach Anspruch 1, dadurch gekennzeichnet, daß man Metallkomplexverbindungen mit einem Reduktionspotential nicht negativer als —1,3 Volt, bezogen auf die Ag/AgCl-Elektrode, verwendet.

3. Verfahren nach Anspruch 1, dadurch gekennzeichnet, daß man Metallkomplexverbindungen mit organischen Liganden verwendet.

4. Verfahren nach Anspruch 1, dadurch gekennzeichnet, daß man solche Metallkomplexverbindungen verwendet, die als Zentralatom $Rh^{I}$, $Rh^{III}$, $Ru^{I}$, $Ru^{III}$, $Ir^{I}$, $Ir^{III}$, $Fe^{II}$, $Fe^{0}$, $Ni^{II}$, $Ni^{0}$, $Co^{III}$, Co und als Liganden 2,2′-Bipyridin, 4,4′-Dimethyl-2,2′-bipyridin, 1,10-Phenanthrolin, 2,2′,6′,2″-Terpyridin, Tetraazamakrocyclen, ein Porphyrin, ein Phthalocyanin oder NO enthalten.

## Claims

1. A process for the electrochemical hydrogenation of nicotinamide adenine dinucleotide ($NAD^{\oplus}$) to NADH in aqueous solution and in the presence of an electron carrier, wherein a metal complex is used as the electron carrier.

2. A process as claimed in claim 1, wherein the metal complex used has a reduction potential which is not more negative than —1.3 volt, based on the Ag/AgCl electrode.

3. A process as claimed in claim 1, wherein the metal complex used possesses organic ligands.

4. A process as claimed in claim 1, wherein the metal complex used contains, as the central atom, $Rh^{I}$, $Rh^{III}$, $Ru^{I}$, $Ru^{III}$, $Ir^{I}$, $Ir^{III}$, $Fe^{II}$, $Fe^{0}$, $Ni^{II}$, $Ni^{0}$, $Co^{III}$, $Co^{I}$ and, as ligands, 2,2′-bipyridine, 4,4′-dimethyl-2,2′-bipyridine, 1,10-phenanthroline, 2,2′,6′,2″-terpyridine, a tetraazamacrocyclic structure, a porphyrin, a phthalocyanine or NO.

## Revendications

1. Procédé pour l'hydrogénation électrochimique de nicotinamide-adénine-dinucléotide ($NAD^{\oplus}$) en NADH, en solution aqueuse et en présence d'un transporteur d'électrons, caractérisé en ce qu'on utilise, comme transporteurs d'électrons, des composés complexes métallifères.

2. Procédé selon la revendication 1, caractérisé en ce qu'on utilise des composés complexes métallifères ayant un potentiel de réduction qui n'est pas plus négatif que —1,3 V par rapport à l'électrode à l'Ag/AgCl.

3. Procédé selon la revendication 1, caractérisé en ce qu'on utilise des composés complexes métallifères comportant des coordinats organiques.

4. Procédé selon la revendication 1, caractérisé en ce qu'on utilise des composés complexes métallifères qui contiennent, en tant qu'atome central, $Rh^{I}$, $Rh^{III}$, $Ru^{I}$, $Ru^{III}$, $Ir^{I}$, $Ir^{III}$, $Fe^{II}$, $Fe^{0}$, $Ni^{II}$, $Ni^{0}$, $Co^{III}$, $Co^{I}$ et, en tant que coordinat, la 2,2′-bipyridine, la 4,4′-diméthyl-2,2′-bipyridine, la 1,10-phénanthroline, la 2,2′,6′,2″-terpyridine, le tétra-azamacrocyclène, une porphyrine, une phthalocyanine ou NO.